# EUROPEAN PATENT APPLICATION

(11) **EP 1 803 458 A1**
(43) Date of publication of application: **04.07.2007**
(21) Application number: 06446002.5
(22) Date of filing: 01.11.2006
(51) Int. Cl.: A61K 33/00, A61P 29/00, A61P 37/08, A61K 31/352, A61K 45/06

(54) **Use of inorganic salts in the treatment of inflammation**

(30) Priority: 23.12.2005 US 318288; 09.06.2006 US 450603
(71) Applicant: Cordray, Scott, Sapulpha OK 74066 (US)
(72) Inventor: Cordray, Scott, Sapulpha OK 74066 (US)
(74) Representative: Karlsson, Leif Karl Gunnar

(57) **Abstract**

The present invention relates to the treatment of inflammation and irritation with non-steroidal compositions containing inert salts including magnesium and potassium salts. More particularly the compositions are used to treat mucosal parts and to provide oral hygiene.

## Description

### Field of the Invention

The present invention relates to non-steroidal anti-inflammatory compositions for treating various inflammations and irritations. More particularly, there is provided a composition of inorganic salts which can be used over a long period of time to reduce inflammation and remove irritants in mucousal cavities as well as the eyes and ears and improving oral hygiene,

### Background of the Invention

Ocular irritations can result from various ophthalmic surgical procedures, contact lens wear, exposure to allergens, ocularly irritating chemical, pollutants, dust particles, ultraviolet light, including various pathogens that cause conjunctivitis.

Ear infections and irritations results from exposure to pathogens while swimming, ear wax build up, insects, and exposure to chemical vapors.

Prompt prophylaxis treatment by ear and eye washes will aid in reducing the length and severity of any irritation.

U.S. Pat. No. 5110493 to Chemg-Chyi et al relates to ophthalmic non-steroidal anti-inflammatory drug formulations containing a quaternary ammonium preservative and a non-ionic surfactant.

There are a number of patents that address various ophthalmic formulations to ease ocular irritations, for example, U.S. Pat. Nos. 5,895,645; 5,877,154; and 5,872,086.

Allergic rhinitis, sinusitis, and epistaxis, as well as common irritations, can each be induced by any one of smoke, chemicals, pollens, mold or dust mites. Pollens cause the release of serine proteases such as tryptase and chymase and the activation of IgE which causes the degranulation of mast cells so as to initiate the acute inflammatory cycle. Mast cells release histamines release factors. Through a number of mechanisms the results can be itchy nose, rhinorrhea and itchy and watery eyes. Inflammation by smoke or chemicals can cause the release of arachadonic acid as well as leukotriene B4. Dust mites are known to increase the amount of eosinophiles in asthma patients. Steroids such as triamcinolone acetonide have been commonly used to treat the symptoms. However, there are problems associated with prolonged use of steroids. More particularly, steroid use by children has been discouraged because nasal sprays will cause swallowing by children so that side effects may result. The use of anti-histamines does not solve the inflammation resulting from mast cell degranulation and cannot be used over prolonged periods.

A variety of microorganisms are also present in the oral cavity which cause inflammation. These range from the natural flora of the host to pathogenic species. Among these microorganisms are the gram-positive bacteria associated with the formation of plaque (a dense, enamel adherent, microorganism-containing polysaccharide matrix). Even with good oral hygiene, it has been shown that microorganisms (including those responsible for plaque formation) rapidly build up in the oral cavity. Specific areas, including periodontal and subgingival spaces harbor bacteria which can lead to plaque and calculus that in turn lead to periodontal and inflammatory disease.

### Summary of the Invention

There is provided compositions and methods for treating the symptoms associated with the irritation and inflammation of oral and mucosal cavities caused by allergens and irritants which cause the release of histamine, arachadonic acid and serine proteases. More particular, there is provided a method for treating irritations and inflammations with a composition comprising:
a) about 1 to 10 percent by weight of salts comprising
   1) about 45 to 60% by weight of magnesium chloride,
   2) about 29 to 41% by weight, of potassium chloride, and
   3) about 1 to 5% by weight of salts selected from the group consisting of magnesium bromide, calcium chloride, calcium bromide, sodium bromide, magnesium sulfate, and sodium chloride,
b) the remainder being water.

The composition is buffered to a pH of about 4.0 to 8.0.

The composition may include plant extracts especially for irrigation and as a decongestant.

It is further advantageous to include in the composition cromolyn, which is a PAR-2 inhibitor.

It is therefore an object of the invention to provide a method and composition for the treatment of irritations and inflammations of the oral cavities.

It is a further object of the invention to provide a hypertonic solution.

It is another object of the invention to reduce optic and otic allergen and irritants by irrigation with a hypertonic aqueous composition comprising inert salts.

It is yet another object of the invention to reduce biofilm and treat gingivital inflammation with a mouthwash.

### Description of the Preferred Embodiments

The present invention relates to a method and composition for treating a patient suffering from an irritation and/or inflammations of the mucosal and oral cavities including optic and otic. More particularly, there is provided a method and composition of administering an effective amount of an aqueous composition comprising:
A) about 1 to 10% by weight, preferably about 1 to 5% by weight of salts comprising:
   1) about 45 to 60% by weight of magnesium chloride, preferably about 55 to 58%.
   2) about 29 to 41 % by weight of potassium chloride, preferably about 39 to 40%,
   3) about 0.4 to 5% by weight of salts selected from the group consisting of magnesium bromide, calcium chloride, calcium bromide, sodium bromide and magnesium sulfate, and
B) the remainder water,
the composition may be buffered to a pH of about 4.0 to 8.0, whereby the irritation and/or inflammation is reduced.

The buffer can comprise a buffer selected from the groups comprising sodium phosphate, potassium phosphate, sodium carbonate and the like as is commonly used by those skilled in the art.

The aqueous composition may include cromolyn in an amount of about 0.5 to 4%, preferably 0.5 to 1%, to further inhibit the degranulation of mast cells and also inhibit the activation of arachadonic acid caused by irritants.

Zinc ions, such as in the form of zinc gluconate, have been found useful when viruses are involved and can be used in amounts up to 2%.

Plant extract can be included such as echinacea which has been reported to inhibit gram-negative bacteria, Myrrh oil from cammphora abyssinica which helps to fight inflammation.

A preferred 100 ml composition of the present invention comprises:

| **Ingredient** | **Wt.** |
|---|---|
| Magnesium Chloride | 1.0 - 2.00g |
| Magnesium Bromide | 0.01 - 0.05 |
| Magnesium Sulfate | 0.0 1 - 0.05 |
| Potassium Chloride | 0.8 - 1.00g |
| Calcium Chloride | 0 - 0.05g |
| Sodium Carbonate | 0 - 0.05g |
| 1% Saline Solution | q.s. |

| | |
|---|---|
| Optionally about 0.1g of cromolyn may be added. | |

Other non-steroidal anti-inflammatory agents which may be included in the formulations are analgesic/nonsteroidal anti-inflammatory compounds. Preferably the non-steroidal anti-inflammatory agent is one or more of the following: aspirin, benoxaprofen, benzofenac, bucloxic acid, butibufen, carprofen, cicloprofen, cinmetacin, cildanac, clopirac, diclofenac, etodolac, fenbufen, fenclofenac, fenclorac, fenoprofen, fentiazac, flunoxaprofen, furaprofen, flurbiprofen, furobufen, furofenac, ibuprofen, ibufenac, indomethacin, indoprofen, isoxepac, ketroprofen, lactorolac, lonazolac, metiazinic, miroprofen, naproxen, oxaprozin, oxepinac, phenacetin, pirprofen, pirazolac, protizinic acid, sulindac, suprofen, tiaprofenic acid, tolmetin, and zomepirac. Preferably, the agent is selected from the group consisting of diclofenac, suprofen, and flurbiprofen sodium and mixtures thereof.

The compositions may contain water soluble polymers or water insoluble polymers as suspending agents. Examples of such soluble polymers are cellulosic polymers like hydroxypropyl methylcellulose. Water insoluble polymers are preferably crosslinked carboxy-vinyl polymers. It is important to note, however, that the present invention may require one of the active ingredients to be in suspension without reference to whether the polymer is or is not in suspension.

A preferred form of the invention incorporates insoluble polymers to provide a gel or liquid drops which release the drug over time. Preferably, the polymer is about 0.1 to 6.5%, more preferably about 1.0 to about 1.3% by weight based on the total weight of the suspension of a crosslinked carboxy-containing polymer. Suitable carboxy-containing polymers for use in the present invention and method for making them are described in U.S. Pat. No. 5,192,535 to Davis et al. which is hereby incorporated by reference and relied upon. These polymer carriers include lightly crosslinked carboxy-containing polymers (such as polycarbophil, or Carbopols. RTM.), dextran, cellulose derivatives, polyethylene glycol 400 and other polymeric demulcents such as polyvinylpyrolidone, polysaccharide gels and Gelrite. RTM.. A carboxy-containing polymer system known by the tradename DuraSite. RTM., containing polycarbophil, is a sustained release topical ophthalmic delivery system that releases the drug at a controlled rate, may also be used.

Aqueous mixtures of this invention may also contain amounts of suspended lightly crosslinked polymer particles ranging from about 0.1% to about 6.5% by weight, and preferably from about 0.5% to about 4.5% by weight, based on the total weight of the aqueous suspension. They will preferably be prepared using pure, sterile water, preferably deionized or distilled, having no physiologically or ophthalmologically harmful constituents, and will be adjusted to a pH of from about 4.0 to 8.0, and preferably from about 5,5 to about 6.5, using any physiologically and ophthalmologically acceptable pH adjusting acids, bases or buffers, e.g., acids such as acetic, boric, citric, lactic, phosphoric, hydrochloric, or the like, bases such as sodium hydroxide, sodium phosphate, sodium borate, sodium citrate, sodium acetate, sodium lactate.

When formulating the aqueous ophthalmic compositions in some cases, their osmotic pressure (.pi.) may be adjusted to from about 10 milliosmolar (mOsM) to about 400 mOsM, using appropriate amounts of physiologically and ophthalmologically acceptable salts. Sodium chloride is preferred to approximate physiologic fluid, and amounts of sodium chloride ranging from about 0.01% to about 1% by weight, and preferably from about 0.05% to about 0.45% by weight, based on the total weight of the aqueous suspension, will give osmolalities within the above-stated ranges. Equivalent amounts of one or more salts made up of cations such as potassium, ammonium and the like and anions such as chloride, citrate, ascorbate, borate, phosphate, bicarbonate, sulfate, thiosulfate, bisulfate, sodium bisulfate, ammonium sulfate, and the like can also be used in addition to or instead of sodium chloride to achieve osmolalities within the above-stated ranges. Sugars like mannitol, dextrose, glucose or other polyols may be added to adjust osmolarity. However, in some instances hypertonic compositions may be required.

When water soluble polymers are used, such as hydroxypropyl methylcellulose, the viscosity will typically be about 10 to about 400 centipoises, more typically about 10 to about 200 centipoises or about 10 to about 25 centipoises.

It is preferred to include a preservative, for example, Thimerosal or benzalkonium chloride and/or an antioxidant, for example, vitamin E. Other filler materials which can be included and are commonly found in nasal compositions as long as the composition remains hypertonic.

The compositions are generally administered every four to eight hours and/or as conditions of the patient and atmosphere require.

The following examples further illustrate the practice of this invention, but are not intended to be limiting thereof. It will be appreciated that the selection of actual amounts of composition to be administered to any individual patient will fall within the discretion of the attending physician and will be prescribed in a manner commensurate with the appropriate dosages will depend on the stage of the disease and like factors uniquely within the purview of the attending physician.

In order that those skilled in the art can more fully appreciate aspects of this invention, the following examples are set forth. These examples are given solely for purposes of illustration and should not be considered as expressing limitations.

### Example 1

A preferred 100 ml composition of the present invention comprises

| **Ingredient** | **Wt.** |
|---|---|
| Magnesium Chloride | 1.0 - 2.00 g |
| Magnesium Bromide | 0.01 - 0.05 |
| Magnesium Sulfate | 0.01 - 0.05 |
| Potassium Chloride | 0.8 - 1.00 g |
| Calcium Chloride | 0 - 0.05 g |
| Sodium Carbonate | 0 - 0.05 g |
| 1% Saline Solution | q.s. |

The pharmaceutical compositions may be prepared for administration to mucosal parts according to standard formulating procedures.

The salts may be dissolved in sterile water, physiological saline solution or buffer solution with a pH of 4.0 to 8.0 which is advantageously ionically balanced.

### Example 2

A 100 ml hypertonic solution is prepared by admixing the following:

| **Ingredient** | **Wt.-gm** |
|---|---|
| Magnesium Chloride | 2.00 |
| Magnesium Bromide | 0.05 |
| Magnesium Sulfate | 0.05 |
| Potasium Chloride | 1.00 |
| Calcium Chloride | 0.05 |
| Sodium Carbonate | 0.05 |
| 1% Saline Solution | q.s. |

The composition can be used to treat optic or otic irritations.

### Example 3

Forty-two patients were treated with either Ringer's lactate hypertonic saline (n=20) or the hypertonic solution of Example 3 for a four week period.

Rhinitis symptom scores based on the severity (0 = no discomfort to 3=severe discomfort) of 16 individual symptoms were totaled to obtain composite scores for baseline (prior to initiating treatment) and after 4 continuous weeks of treatment. The mean baseline symptom score for the hypertonic saline group was 14.9 ± 6.9 versus 17.4 ± 8.4 for the group of Example 3 solution. There was no difference between these values (P=,312). However, after four weeks of treatment, the mean symptom score for the hypertonic saline was 15.2 ± 8.6; this value was not different from baseline (P = .851). In contrast, the mean symptom score for the group of Example 3 solution decreased significantly to 7.7 ± 5.9 (P<.001). The Example 3 solution group mean composite rhinitis symptom score at 4 weeks was significantly lower than the 4-week score in the hypertonic saline patients (P = .003). Twenty-one of 22 patients (95.5%) treated with solution of Example 3 for 4 weeks demonstrated improved mean composite rhinitis scores as compared with 12 of 20 patients (60%) of patients treated with 4 weeks of hypertonic saline.

### Example 4

A mouthwash can be prepared by admixture of the following:
1.30 g of the mixture of Example 1
2.43 g Sucralose
2.28 g Glycerin USP
0.15.25 g Saccharin USP
.0 g. Sodium Fluoride USP
Citric Acid to adjust pH to 4.0

### Example 5

A 100 ml hypertonic solution is prepared by admixing the following:

| **Ingredient** | **Wt. gm** |
|---|---|
| Magnesium Chloride | 2.00 |
| Magnesium Bromide | 0.05 |
| Magnesium Sulfate | 0.05 |
| Potasium Chloride | 1.00 |
| Calcium Chloride | 0.05 |
| Sodium Carbonate | 0.05 |
| 1% Saline Solution | q.s. |

Optionally, the composition can contain 0.5% clove oil and/or zinc gluconate.

### Example 6

Commercially available teeth treatment compositions were tested against biofilm to determine efficacy in removal of biofilm by soaking in a solution for 24 hours.

| **Product** | **Composition** | **Effect** |
|---|---|---|
| 1. BIOVAC^{™} | 0.8 Chlorohexidine, 3.20% EDTA; Proteolytic enzymes Dispersing agent | Slight remove Stain |
| | | |
| 2. EFFERDENT^{™} | Potassium persulfate Sodium borate, Sodium lauryl sulfate, Sodium bicarbonate Magnesium stearate | Slight removal No Stain |
| | | |
| 3. STERISOL | Chlorohexidine, Glycerol, 38F, Alcohol | Slight removal Stain |
| | | |
| 4. Composition of Example 5 | | Complete Removal w/brushing No Stains |

## Claims

1. A method of treating a patient suffering from inflammation and/or irritation which comprises administering to the site of inflammation or irritation an effective amount of an aqueous composition comprising:
A) about 1 to 10% by weight of salts comprising:
1) about 45 to 60% by weight of magnesium chloride;
2) about 29 to 40% by weight of potassium chloride, and
3) about 0.4 to 5% by weight of salts selected from the group consisting of magnesium bromide, calcium chloride, calcium bromide, sodium bromide, sodium chloride and magnesium sulfate,
B) the remainder being water, whereby the irritation or inflammation is reduced.

2. The method of claim 1 wherein said aqueous composition is buffered at about pH 4.0 to 8.0.

3. The method of claim 1 wherein said aqueous composition is hypertonic.

4. The method of claim 3 wherein said hypertonic composition treats eye or ear irritations and is buffered at a pH of 6.0 to 7.5.

5. The method of claim 1 wherein said patient is suffering from nasal inflammation or irritation.

6. The method of claim 5 wherein said patient is suffering from allergic rhinitis.

7. The method of claim 1 wherein said composition is administered to mucosal sites.

8. The method of claim 1 wherein said composition is a mouthwash.

9. The method of claim 1 wherein said composition contains cromolyn.

10. The method of claim 1 wherein said composition includes an antibiotic.

11. The method of claim 9 wherein said composition treats a patient with a periodontal disease.

12. The method of claim 1 wherein said composition has an osmolarity between 140 and 280 mOsm/L.
